# EUROPÄISCHE PATENTSCHRIFT

(11) **EP 1 758 548 B1**
(45) Veröffentlichungstag und Bekanntmachung des Hinweises auf die Patenterteilung: **02.12.2009**
(21) Anmeldenummer: 05759345.1
(22) Anmeldetag: 09.06.2005
(51) Int. Cl.: A61K 8/44, A61Q 5/00, A61Q 15/00, A61Q 19/00

(54) **STABILISIERUNG VON FARBSTOFFEN IN KOSMETISCHEN UND DERMATOLOGISCHEN ZUBEREITUNGEN**
STABILISATION OF COLORANTS IN COSMETIC AND DERMATOLOGICAL PREPARATIONS
STABILISATION DE COLORANTS DANS DES PREPARATIONS COSMETIQUES ET DERMATOLOGIQUES

(30) Priorität: 15.06.2004 DE 102004028629
(43) Veröffentlichungstag der Anmeldung: 07.03.2007
(73) Patentinhaber: BASF SE, 67056 Ludwigshafen (DE)
(72) Erfinder: ANDRE, Valerie, 67063 Ludwigshafen (DE)
(86) Internationale Anmeldenummer: PCT/EP2005/006198
(87) Internationale Veröffentlichungsnummer: WO 2005/123013

(56) Entgegenhaltungen:
- EP-A- 1 291 007
- DE-A1-102004 002 600
- DE-A1-102004 002 602
- PATENT ABSTRACTS OF JAPAN Bd. 1997, Nr. 07, 31. Juli 1997 (1997-07-31) & JP 09 078085 A (KAO CORP), 25. März 1997 (1997-03-25) in der Anmeldung erwähnt

## Beschreibung

Die vorliegende Erfindung betrifft die Farbstabilisierung von kosmetischen und dermatologischen Zubereitungen.

Kosmetische Mittel zeigen während der Lagerung eine gewisse Tendenz zur Zersetzung. Hierbei kann es sich beispielsweise um eine Phasentrennung bei Emulsionspräparaten handeln, um Veränderungen des Viskositätsverhaltens oder um Farb- und Geruchsveränderungen. Insbesondere die Farb- und Geruchsstabilität spielen für die kosmetische Akzeptanz beim Endverbraucher eine bedeutende Rolle. Farbe und Geruch kosmetischer Präparate können sich ändern, wenn die Präparate erhöhten Temperaturen oder Licht ausgesetzt sind, beispielsweise in Schaufensterauslagen.

Um die durch Licht bedingten Veränderungen zu reduzieren, werden kosmetische Mittel daher üblicherweise in nicht-transparente, lichtundurchlässige Behälter abgefüllt. Transparente oder semitransparente Produkt-Behälter werden zur Verbesserung der Lichtstabilität oft aus Materialien gefertigt, denen UV-Filtersubstanzen, wie beispielsweise Benzotriazol, zugesetzt werden. Andererseits können die kosmetischen Mitteln selbst UV-Filtersubstanzen enthalten.

Aus der DE 197 50 906 und der DE 197 39 797 ist beispielsweise der Einsatz von Triazin-Derivaten zur Stabilisierung organischer Materialien gegen UV-Licht, Sauerstoff und erhöhte Temperaturen bekannt. Die EP 0 714 880 betrifft Bismethylenphenylen-Derivate, die nicht nur als Sonnenschutzfaktoren zum Schutz der Haut und Haare Einsatz finden, sondern auch der Verbesserung der Lagerstabilität kosmetischer Zusammensetzungen dienen.

Aus den japanischen Offenlegungsschriften JP 09078085 A und JP 10237488 A sind Haut- und Haarreinigungsmittel bekannt, denen zur Verbesserung der Lagerstabilität bei erhöhter Temperatur Komplexbildner und Antioxidantien zugesetzt werden. Stabilisator-Mischungen aus Antioxidantien und Komplexbildnern werden häufig auch zur Stabilisierung Retinoid-haltiger kosmetischer Präparate verwendet, wie beispielsweise in der EP 0 440 398, WO 96/07396, EP 0 549 592 und EP 0 586 106 beschrieben.

Der gegenwärtige Verbrauchertrend geht zu kosmetischen Produkten in transparenten und semitransparenten Behältern. Aufgabe war es daher, ein Stabilisatorsystem zu entwickeln, das kosmetische Produkte in transparenten oder semitransparenten Behältern gegen die durch Licht bedingten Farb- und Geruchsveränderungen stabilisiert. Die gegenwärtigen Mittel sind in dieser Hinsicht nicht immer ausreichend wirksam.

Diese Aufgabe wurde gelöst durch die Verwendung der aminosubstituierten Hydroxybenzophenonverbindung der Formel I, 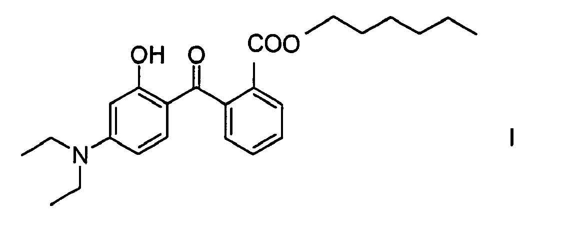 zur Farbstabilisierung von kosmetischen und dermatologischen Zubereitungen.

Die im Rahmen der vorliegenden Erfindung zur Stabilisierung in Betracht kommenden kosmetischen und dermatologischen Zubereitungen enthalten bevorzugt einen oder mehrere organische Farbstoffe. Der organische Farbstoff kann ein wasserlöslicher oder öllöslicher Farbstoff sein oder eine durch sogenannte Verlackung der löslichen Farbstoffe mit Metallionen, vorzugsweise Natrium, Calcium oder Aluminium, erhaltene unlösliche Verbindung sein.

Beispielsweise können die Farbstoffe die aus der entsprechenden Positivliste der Kosmetikverordnung bzw. der EG-Liste kosmetischer Färbemittel ausgewählten Verbindungen sein.

Eine bevorzugte Ausführungsform der erfindungsgemäßen Verwendung ist **dadurch gekennzeichnet, dass** es sich bei den organischen Farbstoffen um Verbindungen ausgewählt aus der Gruppe, bestehend aus Curcumin (E 100), Riboflavin (E 101), Lactoflavin (E 101 a), Tartrazin (E 102), Chinolingelb (E 104), Gelborange S (E 110), Cochenille (E 120), Azorubin (E 122), Amaranth (E 123), Conchenillerot (E 124), Erythrosin (E 127), Rot 2 G (E 128), Allurarot AC (E 129), Patentblau V (E 131), Indigotin I (E 132), Brillantblau FCF (E 133), Chlorophylle (E 140), kupferhaltige Komplexe der Chlorophylle (E 141), Brillantsäuregrün (E 142), Carotinoide (E 160), β-Carotin (E 160a), Annato, Bixin, Norbixin (E 160b), Capsanthin (E 160c), Lycopin (E 160d), β-apo-8-Carotinal (E 160e), β-apo-8-Carotinsäureethylester (E 160e), Xanthophylle (E 161), Lutein (E 161b), Canthaxanthin (E 161g), Beetenrot (E 162) und Anthocyane (E 163) handelt.

Besonders bevorzugte Farbstoffe sind Tartrazin (E 102), Chinolingelb (E 104), Gelborange S (E 110), Conchenillerot (E 124), Indigotin I (E 132).

Erfindungsgemäß wird die Verbindung der Formel I in Konzentrationen von 0,01 bis 5 Gew.-%, bevorzugt von 0,05 bis 3 Gew.-%, besonders bevorzugt in Konzentrationen von 0,08 bis 2 Gew.-%, bezogen auf die Gesamtmenge der kosmetischen oder dermatologischen Zubereitung, eingesetzt.

Eine weitere bevorzugte Ausführungsform der erfindungsgemäßen Verwendung ist **dadurch gekennzeichnet, dass** man die aminosubstituierte Hydroxybenzophenonverbindung der Formel I in einer Mischung zusammen mit dem p-Methoxyzimtsäureester der Formel II einsetzt.

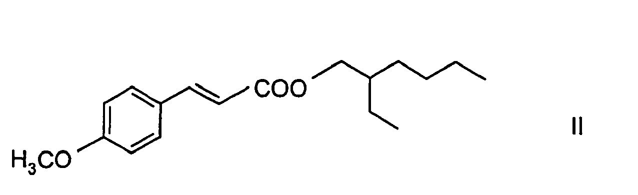

Die Zusammensetzung der Mischung besteht bevorzugt aus 30 bis 70 Gew.-%, besonders bevorzugt aus 30 bis 50 Gew.-%, ganz besonders bevorzugt aus 35 bis 45 Gew.-% der Verbindung I und 70 bis 30 Gew.-%, besonders bevorzugt 70 bis 50 Gew.-%, ganz besonders bevorzugt 65 bis 55 Gew.-% der Verbindung II.

Gegenstand der Erfindung sind auch kosmetische oder dermatologische Zubereitungen, enthaltend mindestens einen organischen Farbstoff und die aminosubstituierte Hydroxybenzophenonverbindung der Formel I.

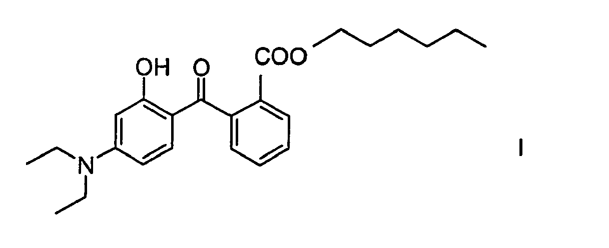

Als bevorzugte organische Farbstoffe der kosmetischen oder dermatologischen Zubereitungen sind Verbindungen, ausgewählt aus der Gruppe, bestehend aus Curcumin (E 100), Riboflavin (E 101), Lactoflavin (E 101a), Tartrazin (E 102), Chinolingelb (E 104), Gelborange S (E 110), Cochenille (E 120), Azorubin (E 122), Amaranth (E 123), Conchenillerot (E 124), Erythrosin (E 127), Rot 2 G (E 128), Allurarot AC (E 129), Patentblau V (E 131), Indigotin I (E 132), Brillantblau FCF (E 133), Chlorophylle (E 140), kupferhaltige Komplexe der Chlorophylle (E 141), Brillantsäuregrün (E 142), Carotinoide (E 160), β-Carotin (E 160a), Annato, Bixin, Norbixin (E 160b), Capsanthin (E 160c), Lycopin (E 160d), β-apo-8-Carotinal (E 160e), β-apo-8-Carotinsäureethylester (E 160e), Xanthophylle (E 161), Lutein (E 161b), Canthaxanthin (E 161g), Beetenrot (E 162) und Anthocyane (E 163) zu nennen.

Besonders bevorzugte Farbstoffe sind Tartrazin (E 102), Chinolingelb (E 104), Gelborange S (E 110), Conchenillerot (E 124), Indigotin I (E 132).

Eine weitere bevorzugte Form der Zubereitungen ist **dadurch gekennzeichnet, dass** sie zusätzlich den p-Methoxyzimtsäureester der Formel II enthält.

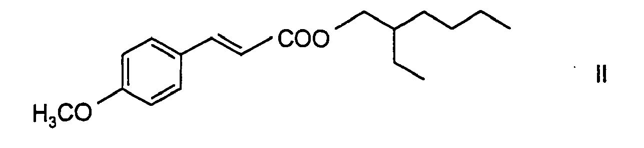

Bei den Zubereitungen kann es sich um kosmetische Reinigungsprodukte wie Schaumbäder, Duschbäder, Reinigungsschäume, Shampoos, um Produkte der Haarpflege wie Haargele und Haarlotionen sowie um Produkte zur Körper- und Hautpflege, wie Cremes, Lotionen, Deos etc. handeln. Die Produkte können auch auf wässriger, wässrig-alkoholischer oder wässrig-glycolischer Basis formuliert werden, und beispielsweise Duftwässer, Gesichtswässer und After-Shaves umfassen. Grundsätzlich liegen hier keine Einschränkungen vor.

Die kosmetischen Zubereitungen enthalten vorzugsweise mindestens ein Tensid aus der Gruppe der Aniontenside, der Niotenside, der amphoteren, der zwitterionischen Tenside und/oder der Kationtenside.

Die Tenside sind in Mengen von 5 bis 40 Gew.-%, bevorzugt 5 bis 20 Gew.-%, jeweils bezogen auf das Gewicht der kosmetischen Zubereitung, enthalten.

Als anionische Tenside eignen sich in erfindungsgemäßen Mitteln alle für die Verwendung am menschlichen Körper geeigneten anionischen oberflächenaktiven Stoffe. Diese sind gekennzeichnet durch eine wasserlöslich machende, anionische Gruppe wie z.B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein.

Beispiele für geeignete anionische Tenside sind, jeweils in Form der Natrium-, Kalium-, Magnesium- und Ammonium- sowie der Mono-, Di- und Trialkanolamrnoniumsalze mit 2 oder 3 C-Atomen in der Alkanolgruppe,
- lineare Fettsäuren mit 10 bis 22 C-Atomen (Seifen),
- Ethercarbonsäuren der Formel R¹-O-(CH₂-CH₂O)ₓ-CH₂-COOH, in der R¹ eine lineare Alkylgruppe mit 10 bis 22 C-Atomen und x = 0 oder 1 bis 16 ist,
- Amidethercarboxylate der Formel [R²-NH(-CH₂-CH₂-O)ₙ-CH₂-COO]ₘZ, in der R² für einen linearen oder verzweigten, gesättigten oder ungesättigten Acylrest mit 2 bis 29 C-Atomen, n für ganze Zahlen von 1 bis 10, m für die Zahlen 1 oder 2 und Z für ein Kation aus der Gruppe der Alkali- oder Erdalkalimetalle steht,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R³-O(-CH₂-CH₂O)ₓ-SO₃H, in der R³ eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- undloder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Kokosmonoglyceridsulfate.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, sowie Sulfobernsteinsäuremono- und -dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Oxyethylgruppen.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R⁴O-(S)ₓ. Diese unterstützen die Milde der erfindungsgemäßen Zubereitungen, haben einen verdickenden Effekt und tragen zu einer verbesserten Solubilisierung der Fettsäurepartialglyceride bei. Sie sind durch die folgenden Parameter gekennzeichnet:

Der Alkylrest R⁴ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl.

Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R⁴ enthalten. Üblicherweise werden diese Verbindungen aber ausgehend von natürlichen Fetten und Ölen oder Mineralölen hergestellt. In diesem Fall liegen als Alkylreste R⁴ Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R⁴
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht-

Als Zuckerbaustein S können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt

Die Alkylglykoside können neben ihrer Tensidwirkung auch dazu dienen, die Fixierung der Duftkomponenten auf dem Haar und der Haut zu verbessern.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkoholund Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder - alkoholate als Katalysatoren verwendet werden. Die Verwendung von Produkten mit eingeengter Homologenverteilung kann bevorzugt sein.

Weiterhin können, insbesondere als Co-Tenside, zwitterionische Tenside verwendet werden. Als zwitterionische Tenside werden solche oberflächenaktiven Verbindungen bezeichnet, die im Molekül mindestens eine quartäre Ammoniumgruppe und mindestens eine -COO⁻- oder -SO₃⁻-Gruppe tragen. Besonders geeignete zwitterionische Tenside sind die sogenannten Betaine wie die N-Alkyl-N,N-dimethylammonium-glycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxylmethyl-3-hydroxyethylimidazolin mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Ikyliminodipropionsäuren, -Hydroxyethyl-N-alkylamidopropylglycine, N-Alkyltaurine, N-alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Beispiele für die kationische Tenside, die vorzugsweise in Haarbehandlungsmitteln verwendet werden, sind insbesondere quartäre Ammoniumverbindungen. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid.

Weiterhin können die sehr gut biologisch abbaubaren quaternären Esterverbindungen, wie beispielsweise die unter dem Warenzeichen Stepantex^{®} vertriebenen Dialkylammoniummethosulfate und Methylhydroxyalkyldialkoyloxyalkylammoniummethosulfate und die entsprechenden Produkte der Dehyquart^{®}-Reihe, als kationische Tenside eingesetzt werden.

Weitere erfindungsgemäß verwendbare kationische Tenside stellen die quaternisierten Proteinhydrolysate dar.

In einer bevorzugten Ausführungsform handelt es sich um einen Reinigungsschaum mit erfindungsgemäßem Stabilisator, der als Tensidkomponenten eine Kombination aus einem Sulfobernsteinsäuremonoestersalz, einem Ampho- oder Betaintensid und einem Alkylpolyglycosid enthält.

In einer besonders bevorzugten Ausführungsform der Erfindung handelt es sich um ein Schaumreinigungsprodukt zur Körper- und Gesichtspflege, bestehend aus einem tensidhaltigen, den Stabilisator enthaltenden Reinigungsmittel.

Abgesehen von dem Stabilisator enthält das kosmetische Produkt in einer bevorzugten Ausführungsform weiterhin wenigstens einen pflegenden Wirkstoff, ausgewählt aus der Gruppe der Vitamine, Provitamine oder Vitaminvorstufen.

Diese sind in den erfindungsgemäßen Mitteln in einer Menge von 0,1-10 Gew.-%, vorzugsweise 0,2-5 Gew.-% und insbesondere 0,5-1 Gew.-%, jeweils bezogen auf das Gewicht der kosmetischen Zubereitung enthalten. Dabei sind erfindungsgemäß solche Vitamine, Pro-Vitamine und Vitaminvorstufen bevorzugt, die üblicherweise den Gruppen A, B, C, F und H zugeordnet werden.

Zur Gruppe der als Vitamin A bezeichneten Substanzen gehören das Retinol (Vitamin Al) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht.

Zur Vitamin B-Gruppe oder Vitamin B-Komplex gehören u.a.
Vitamin B₁ (Thiamin)
Vitamin B₂ (Riboflavin)

Vitamin B₃. Unter dieser Bezeichnung werden häufig die Verbindungen Nicotinsäure und Nicotinsäureamid (Niacinamid) geführt, unter denen insbesondere das Nicotinsäureamid erfindungsgemäß bevorzugt ist.

Vitamin B₅ (Pantothensäure und Panthenol). Im Rahmen dieser Gruppe wird bevorzugt das Panthenol eingesetzt. Erfindungsgemäß einsetzbare Derivate des Panthenols sind insbesondere die Ester und Ether des Panthenols, kationisch derivatisierte Panthenole sowie Pantolacton.

Vitamin B₆ (Pyridoxin sowie Pyridoxamin und Pyridoxal).

Erfindungsgemäß geeignet sind auch Vitamin C (Ascorbinsäure) und dessen Ester, insbesondere Ascorbylpalmitat.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3aS,4S, 6aR)-2-Oxohexahydrothienol-[3,4-d]-imidazol-4-valeriansäure bezeichnet, für die sich aber zwischenzeitlich der Trivialname Biotin durchgesetzt hat.

In einer weiteren bevorzugten Ausführungsform enthält das kosmetische Mittel des erfindungsgemäßen Produktes wenigstens einen Pflanzenextrakt oder ein Destillat aus Pflanzenbestandteilen. Pflanzenextrakte und -destillate steigern häufig die anderen Wirkstoffeigenschaften des Mittels. Die Pflanzenextrakte und -destillate können erfindungsgemäß sowohl in reiner als auch in verdünnter Form eingesetzt werden. Die erfindungsgemäßen Mittel können auch Mischungen aus mehreren verschiedenen Pflanzenextrakten und -destillaten enthalten. Die Pflanzenextrakte und -destillate sind üblicherweise in einer Menge von 0,01-5 Gew.-%, vorzugsweise 0,1-3 Gew.% und insbesondere 0,1-2 Gew.-% Aktivsubstanz, jeweils bezogen auf das Gewicht der kosmetischen Zubereitung, enthalten.

Üblicherweise werden diese Extrakte und -destillate durch Extraktion der gesamten Pflanze bzw. durch Wasserdampfdestillation gewonnen. Es kann aber in einzelnen Fällen auch bevorzugt sein, die Extrakte und Destillate ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (JKW), Frankfurt, beginnenden Tabelle aufgeführt sind.

Erfindungsgemäß sind vor allem die Extrakte und Destillate aus Grünem Tee, Eichenrinde, Brennnessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Erfindungsgemäß bevorzugt sind Mandelextrakte und Destillate aus Hamamelis und Salbei.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt.

Pflanzenextrakte auf Basis von Wasser/Propylenglykol im Verhältnis 1:10 bis 10:1 haben sich als besonders geeignet erwiesen.

Bevorzugt enthalten die erfindungsgemaßen Mittel weiterhin mindestens ein organisches Verdickungsmittel. Solche Verdickungsmittel sind beispielsweise Verdickungsmittel wie Agar-Agar, Guar-Gum, Alginate, Celluloseether, Gelatine, Pektine und/oder Xanthan-Gum. Ethoxilierte Fettalkohole, insbesondere solche mit eingeschränkter Homologenverteilung, wie sie beispielsweise als Handelsprodukt unter der Bezeichnung Arlypon^{®}F (Henkel) auf dem Markt sind, alkoxylierte Methylglucosidester, wie das Handelsprodukt Glutamate^{®} DOE 120 (Amerchol), und ethoxylierte Propylenglykolester, wie das Handelsprodukt Antil^{®} 141 (Goldschmidt) können bevorzugte organische Verdickungsmittel sein.

Als konditionierende Wirkstoffe geeignete Silikonöle und Silikon-Gums sind insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200 und DC 1401 vertriebenen Produkte sowie das Handelsprodukt Fancorsil^{®} LIM-1. Ein geeignetes anionisches Silikonöl ist das Produkt Dow Corning^{®} I784.

Bevorzugt enthaltene pflanzliche Öle und Wachse sind Nachtkerzenöl, Jojobaöl, Sonnenblumenöl, Orangenöl, Mandelöl, Weizenkeimöl und Pfirsichkernöl. Nachtkerzenöl ist besonders bevorzugt.

Insbesondere für die Formulierung sehr milder kosmetischer Zubereitungen hat es sich weiterhin als vorteilhaft erwiesen, wenn die Menge an gelösten anorganischen Salzen auf weniger als 2 Gew.-%, insbesondere weniger als 0,5 Gew.-% begrenzt wird. Dabei ist auch zu beachten, dass solche Salze nicht nur z.B. zur Einstellung der Viskosität zugegeben werden, sondern auch durch andere Wirkstoffe, insbesondere Tenside, eingebracht werden können.

Weitere übliche Bestandteile für die erfindungsgemäßen Mittel sind:
- nichtionische Polymere wie beispielsweise Vinylpyrrolidon/Vinylacrylat-Copolymere, Polyvinylpyrrolidon und Vinylpyrrolidon/Vinylacetat-Copolymere,
- anionische Polymere, wie Polyacryl- und Polymethacrylsäuren, deren Salze, deren Copolymere mit Acrylsäure- und Methacrylsäureestern und -amiden und deren Derivate, die durch Kreuzvernetzung mit polyfunktionellen Agentien erhalten werden,
- Polyoxycarbonsäuren, wie Polyketo- und Polyaldehydocarbonsäuren und deren Salze, sowie Polymere und Copolymere der Crotonsäure mit Estern und Amiden der Acryl- und der Methacrylsäure, wie Vinylacetat-Crotonsäure- und Vinylacetat-Vinylpropionat-Crotonsäure-Copolymere,
- Strukturanten wie Glucose und Maleinsäure,
- haarkonditionierende Verbindungen wie Phospholipide, beispielsweise Sojalecithin, Ei-Lecithin und Kephaline,
- Parfümöle, insbesondere solche mit der Duftnote einer Frucht, wie beispielsweise von Apfel, Birne, Erdbeere, Pfirsich, Aprikose, Ananas, Banane, Kirsche, Kiwi, Mango, Kokos, Mandel, Grapefruit, Maracuja, Mandarine und Melone, oder der Duftnote eines Genußmittels, wie beispielsweise von Tabak, Cola, Kaugummi, Guarana, Schokolade, Kakao, Vanille, Sarsaparilla, Pfefferminze und Rum.
- Lösungsvermittler, wie Ethanol, Isopropariol, Ejhylenglykol, Propylenglykol, Glycerin, Diethylenglykol und ethoxylierte Triglyceride,
- Dimethylisosorbid und Cyclodextrine,
- Farbstoffe,
- Lichtschutzmittel,
- Fette und Wachse, wie Walrat, Bienenwachs, Montanwachs, Paraffine, Ester,
- Antischuppenwirkstoffe wie Climbazol, Piroctone Olamine und Zink Omadine,
- Wirkstoffe wie Bisabolol und Allantoin,
- Konsistenzgeber wie Zuckerester, Polyolester oder Polyolalkylether,
- Glyceride und Fettalkohole,
- Fettsäurealkanolamide,
- Quell- und Penetrationsstoffe wie PCA, Glycerin, Propylenglykolmonoethylether, Carbonate, Hydrogencarbonate, Guanidine, Harnstoffe sowie primäre, sekundäre und tertiäre Phosphate,
- Trübungsmittel wie Latex oder Styrol/Acrylamid-Copolymere,
- Perlglanzmittel wie Ethylenglykolmono- und -distearat oder PEG-3-distearat,
- direktziehende Farbstoffe
- Antioxidantien,
- Konservierungsmittel,
- Treibmittel wie Propan-Butan-Gemische, N20, Dimethylether, CO2 und Luft sowie
- Bitterstoffe, wie beispielsweise Denatonium Benzoate,

Die nachfolgenden Beispiele sollen den Erfindungsgegenstand näher erläutern, ohne ihn hierauf zu beschränken.

### Beispiele für kosmetische Formulierungen

### Bespiel 1

### Haarwasser:

| % | Rohstoff | Ansatz (400 g) |
|---|---|---|
| Phase A | | |
| 40,0 | Ethanol abs. | Alcohol |
| 2,0 | Cremophor CO 40 | PEG-40 Hydrogenated Castor Oil |
| 0,1 | Bisabolol, rac. | Bisabolol |
| 0,1 | Verbindung I | |
| | | |

| Phase B | | |
|---|---|---|
| 2,0 | Luviquat FC 550 | Polyquaternium-16 |
| 1,0 | Farbstofflösung (Chinolingelb) 1 % in Wasser | |
| 54,8 | Wasser, demin. | Water |
| | | |

### Beispiel 2

### Shampoo:

| % | Rohstoff | Ansatz (400 g) |
|---|---|---|
| Phase A | | |
| 40,0 | Texapon NSO | Sodium Laureth Sulfate |
| 5,0 | Tego Betain HS | Cocamidopropyl Batein, Glyceryl Laurate |
| 1,0 | Comperlan KD | Cocamide DEA |
| 5,0 | Luviquat FC 550 | Polyquaternium-16 |
| 1,0 | Luviquat Mono CP | Hydroxyethyl Cetyldimonium Phosphate |
| 0,1 | Euxyl K 100 | Benzyl Alcohol, Methylchloroisothiazolinone, Methylisothiazolinone |
| 0,1 | Verbindung I | |
| 0,1 | Edeta BD | Disodium EDTA |
| 1,0 | Farbstofflösung (Chinolingelb) 1 % in Wasser | |
| 54,8 | Wasser, demin. | Water |
| | | |

### Bespiel 3

### Haarwasser:

| % | Rohstoff | Ansatz (400 g) |
|---|---|---|
| Phase A | | |
| 40,0 | Ethanol abs. | Alcohol |
| 2,0 | Cremophor CO 40 | PEG-40 Hydrogenated Castor Oil |
| 0,1 | Bisabolol, rac. | Bisabolol |
| 0,1 | Mischung aus Verbindung I (35 Gew.-%) und Verbindung II (65 Gew.-%) | |

| Phase B | | |
|---|---|---|
| 2,0 | Luviquat FC 550 | Polyquaternium-16 |
| 1,0 | Farbstofflösung (Chinolingelb) 1% in Wasser | |
| 54,8 | Wasser, demin. | Water |
| | | |

### Beispiel 4

### Shampoo:

| % | Rohstoff | Ansatz (400 g) |
|---|---|---|
| Phase A | | |
| 40,0 | Texapon NSO | Sodium Laureth Sulfate |
| 5,0 | Tego Betain HS | Cocamidopropyl Batein, Glyceryl Laurate |
| 1,0 | Comperlan KD | Cocamide DEA |
| 5,0 | Luviquat FC 550 | Polyquaternium-16 |
| 1,0 | Luviquat Mono CP | Hydroxyethyl Cetyldimonium Phosphate |
| 0,1 | Euxyl K 100 | Benzyl Alcohol, Methylchloroisothiazolinone, Methylisothiazolinone |
| 0,1 | Mischung aus Verbindung I (35 Gew.-%) und Verbindung II (65 Gew.-%) | |
| 0,1 | Edeta BD | Disodium EDTA |
| 1,0 | Farbstofflösung (Chinolin-gelb) 1% in Wasser | |
| 54,8 | Wasser, demin. | Water |
| | | |

## Patentansprüche

1. Verwendung der aminosubstituierten Hydroxybenzophenonverbindung der Formel I, 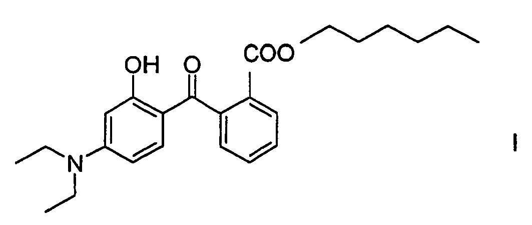 zur Farbstabilisierung von kosmetischen und dermatologischen Zubereitungen.

2. Verwendung nach Anspruch 1, **dadurch gekennzeichnet, dass** die kosmetischen und dermatologischen Zubereitungen organische Farbstoffe enthalten.

3. Verwendung nach einem der Anspruch 2, **dadurch gekennzeichnet, dass** es sich bei den organischen Farbstoffen um wasserlösliche Farbstoffe, öllösliche Farbstoffe oder wasserunlösliche Farblacke handelt.

4. Verwendung nach einem der Ansprüche 1 bis 3, **dadurch gekennzeichnet, dass** es sich bei den organischen Farbstoffen um Verbindungen ausgewählt aus der Gruppe, bestehend aus Curcumin (E 100), Riboflavin (E 101), Lactoflavin (E 101 a), Tartrazin (E 102), Chinolingelb (E 104), Gelborange S (E 110), Cochenille (E 120), Azorubin (E 122), Amaranth (E 123), Conchenillerot (E 124), Erythrosin (E 127), Rot 2 G (E 128), Allurarot AC (E 129), Patentblau V (E 131), Indigotin I (E 132), Brillantblau FCF (E 133), Chlorophylle (E 140), kupferhaltige Komplexe der Chlorophylle (E 141), Brillantsäuregrün (E 142), Carotinoide (E 160), β-Carotin (E 160a), Annato, Bixin, Norbixin (E 160b), Capsanthin (E 160c), Lycopin (E 160d), β-apo-8-Carotinal (E 160e), β-apo-8-Carotinsäureethylester (E 160e), Xanthophylle (E 161), Lutein (E 161b), Canthaxanthin (E 161g), Beetenrot (E 162) und Anthocyane (E 163) handelt

5. Verwendung nach einem der Ansprüche 1 bis 4, **dadurch gekennzeichnet, dass** man die Verbindung der Formel I in Konzentrationen von 0,01 bis 5 Gew.-%, bezogen auf die Gesamtmenge der kosmetischen oder dermatologischen Zubereitung, einsetzt.

6. Verwendung nach einem der Ansprüche 1 bis 5, **dadurch gekennzeichnet, dass** man die aminosubstituierte Hydroxybenzophenonverbindung der Formel I in einer Mischung zusammen mit dem p-Methoxyzimtsäureester der Formel II einsetzt.

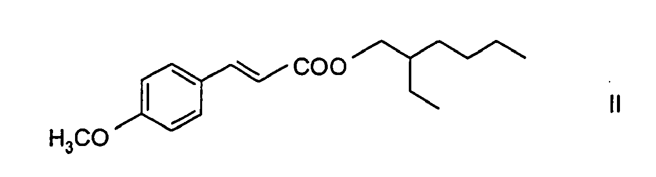

7. Verwendung nach Anspruch 6, **dadurch gekennzeichnet, dass** die Mischung aus 30 bis 70 Gew.-% der Verbindung I und 70 bis 30 Gew.-% der Verbindung II besteht.

8. Kosmetische oder dermatologische Zubereitungen, enthaltend mindestens einen organischen Farbstoff und die aminosubstituierte Hydroxybenzophenonverbindung der Formel I.

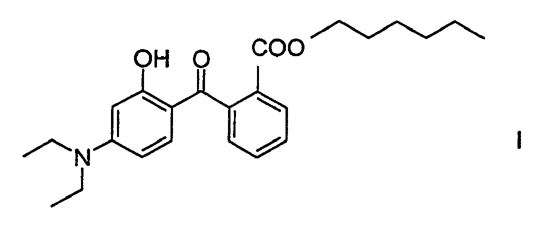

9. Zubereitungen nach Anspruch 8, enthaltend mindestens einen organischen Farbstoff, ausgewählt aus der Gruppe, bestehend aus Curcumin (E 100), Riboflavin (E 101), Lactoflavin (E 101 a), Tartrazin (E 102), Chinolingelb (E 104), Gelborange S (E 110), Cochenille (E 120), Azorubin (E 122), Amaranth (E 123), Conchenillerot (E 124), Erythrosin (E 127), Rot 2 G (E 128), Allurarot AC (E 129), Patentblau V (E 131), Indigotin I (E 132), Brillantblau FCF (E 133), Chlorophylle (E 140), kupferhaltige Komplexe der Chlorophylle (E 141), Brillantsäuregrün (E 142), Carotinoide (E 160), β-Carotin (E 160a), Annato, Bixin, Norbixin (E 160b), Capsanthin (E 160c), Lycopin (E 160d), β-apo-8-Carotinal (E 160e), β-apo-8-Carotinsäureethylester (E 160e), Xanthophylle (E 161), Lutein (E 161b), Canthaxanthin (E 161g), Beetenrot (E 162) und Anthocyane (E 163).

10. Zubereitungen nach einem der Ansprüche 8 oder 9, enthaltend zusätzlich den p-Methoxyzimtsäureester der Formel II.

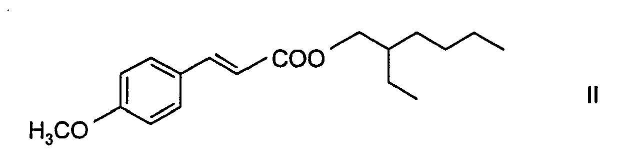

11. Zubereitungen nach einem der Ansprüche 8 bis 10, **dadurch gekennzeichnet, dass** es sich um Shampoos, Haargele, Haarlotionen, Hautcremes, Hautlotionen, Deos, Duftwässer, Gesichtswässer und After-Shaves handelt.

## Claims

1. The use of the amino-substituted hydroxybenzophenone compound of the formula I, 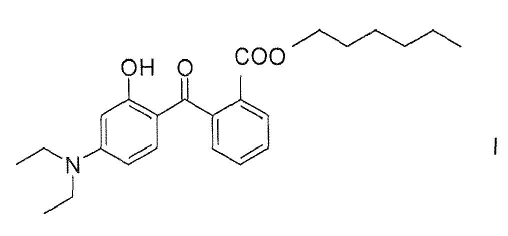 for stabilizing the color of cosmetic and dermatological preparations.

2. The use according to claim 1, wherein the cosmetic and dermatological preparations comprise organic dyes.

3. The use according to claim 2, wherein the organic dyes are water-soluble dyes, oil-soluble dyes or water-insoluble colored lakes.

4. The use according to one of claims 1 to 3, wherein the organic dyes are compounds chosen from the group consisting of curcumin (E 100), riboflavin (E 101), lactoflavin (E 101 a), tartrazine (E 102), quinoline yellow (E 104), yellow orange S (E 110), cochineal (E 120), azorubin (E 122), amaranth (E 123), cochineal red (E 124), erythrosine (E 127), red 2 G (E 128), allura red AC (E 129), patent blue V (E 131), indigo tin I (E 132), brilliant blue FCF (E 133), chlorophylls (E 140), copper-containing complexes of the chlorophylls (E 141), brilliant acid green (E 142), carotenoids (E 160), β-carotene (E 160a), annato, bixin, norbixin (E 160b), capsanthin (E 160c), lycopene (E 160d), β-apo-8-carotenal (E 160e), β-apo-8-carotenic ethyl ester (E 160e), xanthophylls (E 161), lutein (E 161 b), canthaxanthin (E 161g), beetroot (E 162) and anthocyans (E 163).

5. The use according to one of claims 1 to 4, wherein the compound of the formula I is used in concentrations of from 0.01 to 5% by weight, based on the total amount of cosmetic or dermatological preparation.

6. The use according to one of claims 1 to 5, wherein the amino-substituted hydroxybenzophenone compound of the formula I is used in a mixture together with the p-methoxycinnamic ester of the formula II.

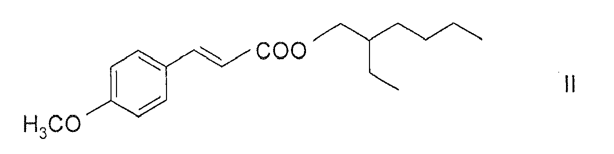

7. The use according to claim 6, wherein the mixture consists of 30 to 70% by weight of the compound I and 70 to 30% by weight of the compound II.

8. A cosmetic or dermatological preparation comprising at least one organic dye and the amino-substituted hydroxybenzophenone compound of the formula I.

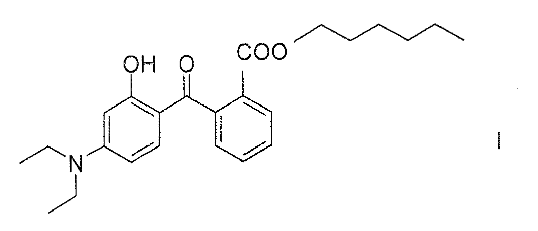

9. The preparation according to claim 8, comprising at least one organic dye chosen from the group consisting of curcumin (E 100), riboflavin (E 101), lactoflavin (E 101 a), tartrazine (E 102), quinoline yellow (E 104), yellow orange S (E 110), cochineal (E 120), azorubin (E 122), amaranth (E 123), cochineal red (E 124), erythrosine (E 127), red 2 G (E 128), allura red AC (E 129), patent blue V (E 131), indigo tin I (E 132), brilliant blue FCF (E 133), chlorophylls (E 140), copper-containing complexes of the chlorophylls (E 141), brilliant acid green (E 142), carotenoids (E 160), β-carotene (E 160a), annato, bixin, norbixin (E 160b), capsanthin (E 160c), lycopene (E 160d), β-apo-8-carotenal (E 160e), β-apo-8-carotenic ethyl ester (E 160e), xanthophylls (E 161), lutein (E 161b), canthaxanthin (E 161g), beetroot (E 162) and anthocyans (E 163).

10. The preparation according to one of claims 8 or 9, additionally comprising the p-methoxycinnamic ester of the formula II.

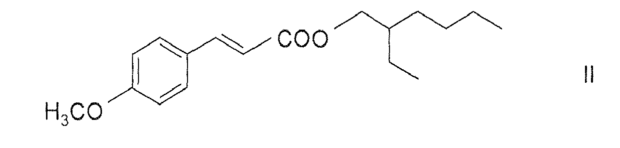

11. The preparation according to one of claims 8 to 10, which is a shampoo, hair gel, hair lotion, skin cream, skin lotion, deodorant, toilet water, face tonic and after-shave.

## Revendications

1. Utilisation du composé hydroxybenzophénone de formule I, 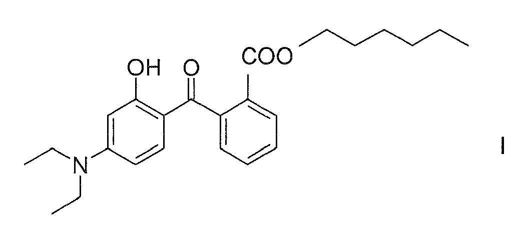 pour la stabilisation de la couleur de préparations cosmétiques et dermatologiques.

2. Utilisation selon la revendication 1, **caractérisée en ce que** les préparations cosmétiques et dermatologiques contiennent des colorants organiques.

3. Utilisation selon la revendication 2, **caractérisée en ce que** les colorants organiques consistent en des colorants hydrosolubles, des colorants liposolubles ou des vernis colorés insolubles dans l'eau.

4. Utilisation selon l'une quelconque des revendications 1 à 3, **caractérisée en ce que** les colorants organiques consistent en des composés choisis dans le groupe constitué par la curcumine (E 100), la riboflavine (E 101), la lactoflavine (E 101a), la tartrazine (E 102), le jaune de quinoléine (E 104), le jaune orangé S (E 110), la cochenille (E 120), l'azorubine (E 122), l'amarante (E 123), le rouge cochenille (E 124), l'érythrosine (E 127), le rouge 2 G (E 128), le rouge Allura AC (E 129), le bleu patenté V (E 131), l'indigotine I (E 132), le bleu brillant FCF (E 133), la chlorophylle (E 140), les complexes cuprifères de la chlorophylle (E 141), le vert acide brillant (E 142), les caroténoïdes (E 160), le β-carotène (E 160a), l'annatto, la bixine, la norbixine (E 160b), la capsanthine (E 160c), le lycopène (E 160d), le β-apo-8-carotinal (E 160e), le β-apo-8-caroténoate d'éthyle (E 160e), la xanthophylle (E 161), la lutéine (E 161b), la canthaxanthine (E 161g), le rouge de betterave (E 162) et les anthocyanes (E 163).

5. Utilisation selon l'une quelconque des revendications 1 à 4, **caractérisée en ce qu'**on utilise le composé de formule I à des concentrations de 0,01 à 5 % en poids, par rapport à la quantité totale des préparations cosmétiques ou dermatologiques.

6. Utilisation selon l'une quelconque des revendications 1 à 5, **caractérisée en ce qu'**on utilise le composé hydroxybenzophénone substitué par amino, de formule I en un mélange conjointement avec l'ester d'acide p-méthoxycinnamique de formule II.

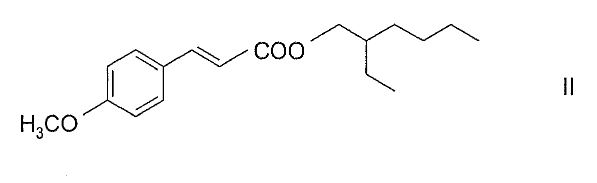

7. Utilisation selon la revendication 6, **caractérisée en ce que** le mélange est constitué de 30 à 70 % en poids du composé I et de 70 à 30 % en poids du composé II.

8. Préparations cosmétiques ou dermatologiques, contenant au moins un colorant organique et le composé hydroxybenzophénone substitué par amino, de formule I.

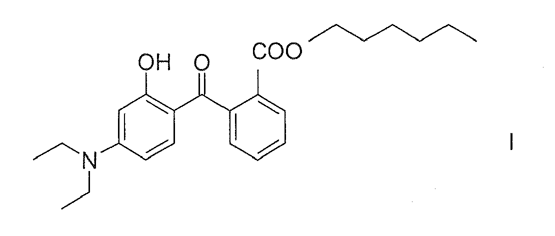

9. Préparations selon la revendication 8, contenant au moins un colorant organique choisi dans le groupe constitué par la curcumine (E 100), la riboflavine (E 101), la lactoflavine (E 101a), la tartrazine (E 102), le jaune de quinoléine (E 104), le jaune orangé S (E 110), la cochenille (E 120), l'azorubine (E 122), l'amarante (E 123), le rouge cochenille (E 124), l'érythrosine (E 127), le rouge 2 G (E 128), le rouge Allura AC (E 129), le bleu patenté V (E 131), l'indigotine I (E 132), le bleu brillant FCF (E 133), la chlorophylle (E 140), les complexes cuprifères de la chlorophylle (E 141), le vert acide brillant (E 142), les caroténoïdes (E 160), le β-carotène (E 160a), l'annatto, la bixine, la norbixine (E 160b), la capsanthine (E 160c), le lycopène (E 160d), le β-apo-8-carotinal (E 160e), le β-apo-8-caroténoate d'éthyle (E 160e), la xanthophylle (E 161), la lutéine (E 161b), la canthaxanthine (E 161g), le rouge de betterave (E 162) et les anthocyanes (E 163).

10. Préparations selon l'une quelconque des revendications 8 et 9, contenant en outre l'ester d'acide p-méthoxycinnamique de formule II.

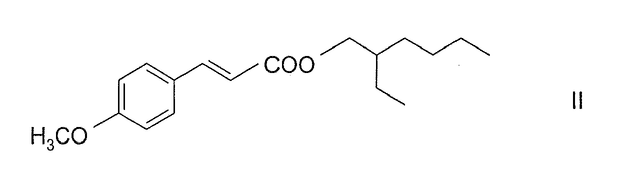

11. Préparations selon l'une quelconque des revendications 8 à 10, **caractérisées en ce qu'**il s'agit de shampooings, de gels capillaires, de lotions capillaires, de crèmes pour la peau, de lotions pour la peau, de déodorants, d'eaux de toilette, de lotions pour le visage et d'après-rasages.
